# FASCICULE DE BREVET EUROPEEN

(11) **EP 2 349 153 B1**
(45) Date de publication et mention de la délivrance du brevet: **23.03.2016**
(21) Numéro de dépôt: 09756036.1
(22) Date de dépôt: 15.10.2009
(51) Int. Cl.: A61F 13/00, A61F 13/06, A61F 13/08

(54) **BANDE DE CONTENTION ET GAMME COMPRENANT PLUSIEURS BANDES DE CONTENTION**
KOMPRESSIONBANDAGE
SUPPORT BAND

(30) Priorité: 31.10.2008 FR 0806068
(43) Date de publication de la demande: 03.08.2011
(73) Titulaire: Gibaud, 42000 Saint Etienne (FR)
(72) Inventeur: BECKERS, Thomas, IS-210 Gardabaer (IS); DUPORT, Guillaume, F-42000 Saint Etienne (FR)
(74) Mandataire: Delorme, Nicolas
(86) Numéro de dépôt international: PCT/FR2009/051971
(87) Numéro de publication internationale: WO 2010/049620

(56) Documents cités:
- EP-A- 0 490 793
- WO-A-94/12133
- WO-A-96/31175
- WO-A-03/005945
- FR-A- 2 544 982
- US-A- 6 050 967

## Description

La présente invention concerne une bande de contention, ainsi qu'une gamme comprenant plusieurs bandes de contention, permettant une adaptation aux différentes morphologies par un système de tailles.

Les bandes de contention sont utilisées pour entourer une partie du corps, notamment la jambe, avec une certaine tension, par exemple dans le cas d'une personne souffrant de problèmes de circulation sanguine.

Il est important que la compression exercée sur la jambe par la bande ne soit pas trop faible, pour que la bande reste en place sur la jambe et puisse jouer son rôle thérapeutique, mais pas trop importante non plus, ce qui conduirait à un effet de garrot non souhaitable. La bande doit apporter la juste compression demandée par le personnel compétent.

A cet effet, on connaît déjà des bandes de contention comprenant des motifs dont la dimension longitudinale est inférieure à la dimension transversale à l'état non contraint de la bande, la dimension longitudinale pouvant être rendue sensiblement égale à la dimension transversale lorsqu'une traction longitudinale adéquate est exercée sur la bande. Avec ce système, on a une indication visuelle du degré de traction appliqué à la bande, et donc de la compression appliquée à la jambe du patient. Il suffit au fabricant de la bande de déterminer les dimensions des motifs en fonction de la nervosité élastique de la bande et de la compression souhaitée de la jambe.

Concrètement, ces bandes de contention connues comprennent des motifs rectangulaires ou ovales, à l'état non contraint de la bande. Ces motifs deviennent respectivement carrés ou ronds lorsque la traction adéquate est exercée. Ces motifs sont réalisés par impression sur la bande à l'état non contraint. Ce procédé d'impression pose un certain nombre de problèmes.

D'une part, lors de l'allongement de la bande, les parties longitudinales des motifs se trouvent étirées, et la zone imprimée a tendance à se craqueler, surtout au fur et à mesure des usages de la bande. En conséquence, la bande devient peu esthétique. De plus, s'ils sont très abîmés, les motifs ne peuvent plus jouer leur rôle de témoin de l'allongement adéquat de la bande.

D'autre part, le fait d'imprimer des zones longitudinales sur la bande de contention peut nuire à son élasticité, les zones imprimées empêchant un allongement libre de la bande.

Par ailleurs, la compression P appliquée au patient suit la loi de Laplace, à savoir P = T / R, où T est la tension appliquée au tissu et R est le rayon de la cheville du patient.

Les bandes connues proposent, pour une même classe de nervosité, une seule tension de bande, qui est atteinte lorsque les motifs ont une forme carrée ou ronde, comme expliqué ci-dessus, et ce quel que soit le tour de cheville du patient. En conséquence, la compression appliquée varie d'un patient à l'autre. Ainsi, puisque T est constante et imposée par les motifs, un patient faisant de la rétention d'eau, et ayant donc une cheville de grand diamètre, aura une compression P moins importante qu'un patient n'ayant pas une cheville gonflée. Or c'est justement le patient dont la cheville est gonflée qui devrait avoir une plus grande compression.

Les bandes connues ne peuvent donc pas s'adapter de façon satisfaisante aux différentes morphologies, pathologiques ou non, des patients.

On connaît par ailleurs du document US 6 050 967 une bande réalisée en un tissu comportant des fils de chaîne et des fils de trame. Cette bande comporte de plus des fils indicateurs qui sont complètement indépendants des fils de chaîne du fond du tissu et insérés par des dispositifs appropriés appelés trameurs. Chaque fil indicateur forme à la fois la portion longitudinale d'un rectangle et une partie de la portion transversale de ce rectangle.

La bande proposée par ce document présente un certain nombre d'inconvénients.

Tout d'abord, une fois que la bande munie de ses fils indicateurs est réalisée, elle subit généralement des opérations d'apprêtage et de fixation thermique pouvant conduire à un retrait. Il s'ensuit que les rectangles dessinés initialement par les fils indicateurs peuvent se retrouver complètement déformés (trop larges ou trop longs). De ce fait, ils ne pourront pas, lors de la mise en place ultérieure de la bande, fournir une indication correcte de la tension appliquée à la bande.

En outre, avec la bande proposée dans le document US 6 050 967, les motifs rectangulaires sont bien distincts lorsque la bande est à l'état non contraint mais, lorsque la bande est étirée, on observe des motifs aux contours peu précis. On n'a alors plus une ligne longitudinale continue, ni une unique ligne transversale bien nette et continue formant un côté commun de deux rectangles adjacents. Or c'est précisément lorsque la bande est tendue, au cours de sa mise en place, que ce caractère continu est utile car il permet de voir distinctement les carrés qui sont des témoins du degré d'allongement approprié de la bande.

La présente invention vise à remédier aux inconvénients mentionnés ci-dessus.

A cet effet, et selon un premier aspect, l'invention concerne une bande du type précité, dans laquelle les motifs sont formés par :
- deux lignes longitudinales s'étendant sur sensiblement toute la longueur de la bande, espacées d'une distance D1, ces lignes longitudinales étant constituées chacune d'au moins un fil de chaîne d'une couleur distincte de la couleur de la bande ;
- et une pluralité de lignes transversales, reliant les deux lignes longitudinales, ces lignes transversales étant sensiblement régulièrement espacées d'une distance D2 inférieure à D1, à l'état non contraint de la bande, et présentant une couleur distincte de la couleur de la bande.

Ainsi, grâce à l'invention, l'allongement longitudinal de la bande, y compris de façon répétée, n'altère pas l'intégrité des lignes longitudinales qui restent donc parfaitement continues et visibles. En outre, l'invention s'affranchit du problème de perte d'élasticité rencontré avec les bandes imprimées de l'art antérieur, comme indiqué ci-dessus. Bien entendu, le fil utilisé pour la réalisation des lignes longitudinales présente lui aussi l'élasticité requise.

Par « couleur distincte », on entend que la couleur des lignes est d'une part différente de la couleur générale de la bande, et d'autre part nettement distinguable de cette couleur générale de la bande. Par exemple, la bande peut être de couleur chair et les lignes longitudinales et/ou transversales d'une couleur qui tranche, tel que le rouge ou le noir. Il est à noter que les lignes ne sont pas nécessairement toutes de la même couleur.

L'invention fournit une bande avec des motifs adjacents continus, non séparés et non espacés les uns des autres.

Un des avantages de l'invention est que, même si les opérations d'apprêtage ou de fixation thermique conduisent à un retrait de la bande, on a toujours deux lignes longitudinales continues et parallèles. La modification éventuelle de la distance entre les lignes longitudinales, due à ces opérations, peut être compensée par l'espacement entre les lignes transversales lorsqu'elles sont réalisées ultérieurement par impression. C'est également de cette manière que peuvent être créées des tailles pour appliquer plus ou moins de contention selon le tour de cheville du patient.

Selon l'invention, les lignes longitudinales sont sensiblement rectilignes et continues, y compris à l'état tendu de la bande, c'est-à-dire lorsqu'une traction longitudinale adéquate est exercée sur la bande.

Selon une réalisation possible, les lignes transversales s'étendent de part et d'autre des deux lignes longitudinales, mais pas forcément jusqu'aux bords longitudinaux de la bande. On a ainsi sur la bande un motif ressemblant à des rails et des traverses de chemin de fer.

Les lignes transversales peuvent être imprimées. En effet, l'impression dans le sens transversal ne présente pas les inconvénients précités de l'impression dans le sens longitudinal. Il est donc possible d'avoir recours à cette technique simple sans nuire à la qualité de la bande de contention.

Les lignes transversales peuvent également être obtenues par un effet d'armure, c'est-à-dire par un mode d'entrecroisement particulier des fils de chaîne et des fils de de trame qui produit des rayures se détachant nettement du fond, aux emplacements appropriés.

En variante, les lignes transversales peuvent être constituées par au moins un fil de trame.

On peut par ailleurs prévoir que la bande de contention présente plusieurs portions selon sa direction longitudinale, chaque portion comportant une pluralité de lignes transversales sensiblement régulièrement espacées d'une distance Di inférieure à D1, les distances Di variant d'une portion à l'autre de la bande. Ainsi, en définissant de manière appropriée la position des portions successives et la distance Di dans chaque portion, on permet à un utilisateur d'appliquer une traction de la bande plus ou moins importante, et calibrée, selon l'endroit du corps où la portion en question sera placée. Par exemple, on peut obtenir une compression plus importante du mollet que du pied dans le cas de la mise en place autour de la jambe.

On peut également prévoir de fournir plusieurs bandes ayant chacune un schéma de répartition des lignes transversales et/ou longitudinales différent.

Selon un deuxième aspect, l'invention concerne une gamme qui comprend N bandes de contention telles que définies précédemment, où N ≥ 2.

Selon un premier mode de réalisation, les lignes longitudinales d'une bande donnée sont espacées d'une distance Dj, les distances Dj étant différentes pour toutes les bandes de la gamme, et les lignes transversales sont espacées d'une distance D2, la distance D2 étant identique pour toutes les bandes de la gamme et inférieure à toutes les distances Dj des bandes de la gamme.

Selon un deuxième mode de réalisation, les lignes longitudinales d'une bande donnée sont espacées d'une distance D1, la distance D1 étant identique pour toutes les bandes de la gamme, et les lignes transversales sont espacées d'une distance Dk, les distances Dk étant différentes pour toutes les bandes de la gamme et inférieures à D1.

Dans l'un ou l'autre de ces cas, on a donc un ensemble de bandes qu'il faut plus ou moins étirer pour rendre les motifs carrés, c'est-à-dire pour atteindre la traction appropriée. Cette différence d'allongement d'application d'une bande à l'autre induira une différence de tension d'application, idéalement étalonnée selon le tour de cheville du patient de manière à procurer une même pression thérapeutique quelle que soit la morphologie du patient.

On décrit à présent, à titre d'exemple non limitatif, un mode de réalisation possible de l'invention, en référence aux figures annexées :
La figure 1 est une vue en plan d'une partie d'une bande de contention selon l'invention, à l'état non contraint ;
La figure 2 est une vue similaire à la figure 1, la bande de contention étant étirée avec la traction appropriée ;
La figure 3 représente la jambe d'une personne autour de laquelle on enroule la bande de contention avec la tension appropriée ;
Les figures 4a et 4b montrent deux bandes d'une même gamme, à l'état non contraint, dont les lignes longitudinales sont espacées respectivement d'une distance Dja et Djb ; et
Les figures 5a et 5b montrent les bandes illustrées respectivement sur la figure 4a et sur la figure 4b, étirées chacune avec la traction appropriée.

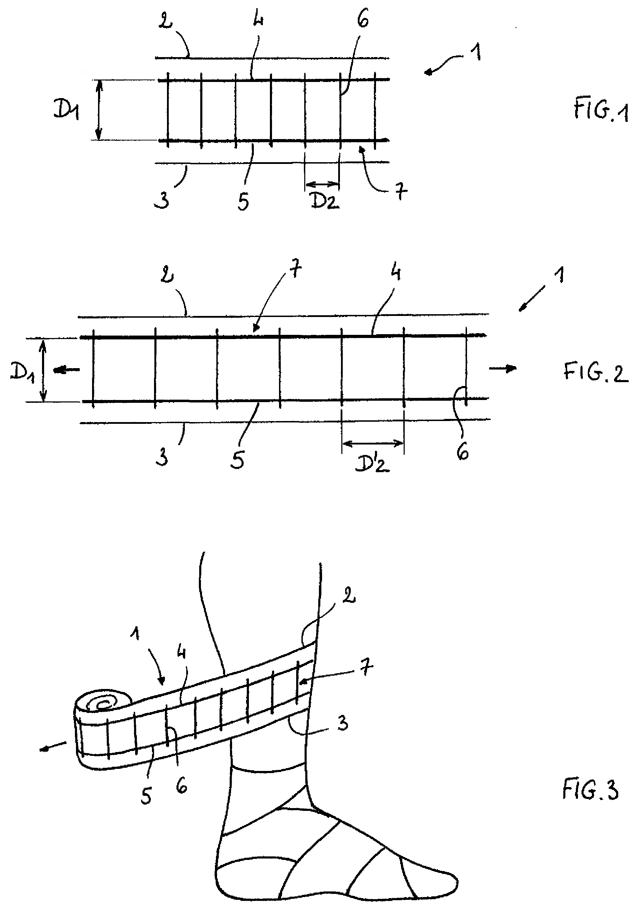

La figure 1 représente une bande de contention 1 destinée à être mise en place, avec une certaine tension, autour d'une partie du corps d'un patient, par exemple autour de la jambe, comme illustré sur la figure 3.

La bande 1 est réalisée par tissage de fils de chaîne et de trame d'une certaine couleur, par exemple couleur chair, et qui présentent l'élasticité et la nervosité requise pour cette application. Elle comporte deux bords longitudinaux 2, 3 sensiblement parallèles.

La bande comporte deux lignes longitudinales 4, 5 parallèles et espacées d'une distance D1, qui sont constituées chacune par au moins un fil de chaîne, par exemple un unique fil de chaîne. Les lignes longitudinales 4, 5 s'étendent sur toute la longueur de la bande 1 et le ou les fils de chaîne qui les constituent présentent une couleur différente de la couleur générale de la bande, de façon que les lignes 4, 5 soient nettement visibles.

En outre, la bande 1 comprend une pluralité de lignes transversales 6 reliant les deux lignes longitudinales 4, 5, et sensiblement régulièrement espacées d'une distance D2 inférieure à D1, à l'état non contraint de la bande 1. Ces lignes transversales 6 présentent également une couleur distincte de la couleur de la bande 1, pour être nettement visibles.

Dans la réalisation représentée, les lignes transversales 6 s'étendent de part et d'autre des deux lignes longitudinales 4, 5, mais pas jusqu'aux bords longitudinaux 2, 3 de la bande 1, et sont réalisées par impression sur la bande 1 à l'état non contraint.

Comme illustré sur la figure 1, à l'état non contraint, la bande 1 comporte ainsi des motifs rectangulaires 7.

Lorsqu'on allonge la bande 1 dans la direction longitudinale, comme illustré sur la figure 2, on provoque d'une part l'allongement des lignes longitudinales 4, 5, tout en conservant la continuité visuelle de ces lignes, et d'autre part l'espacement des lignes transversales 6. Lorsque l'écartement D'2 entre deux lignes transversales 6 successives devient sensiblement identique à D1, l'applicateur sait qu'il a atteint l'allongement, et donc la tension adéquate, qui lui fournira le niveau de compression requis pour soigner la pathologie du patient.

La figure 3 illustre la mise en place de la bande 1, initialement enroulée, sur la jambe d'un patient. La flèche montre la traction exercée sur la bande pour l'obtention requise de motifs 7 carrés et non plus rectangulaires.

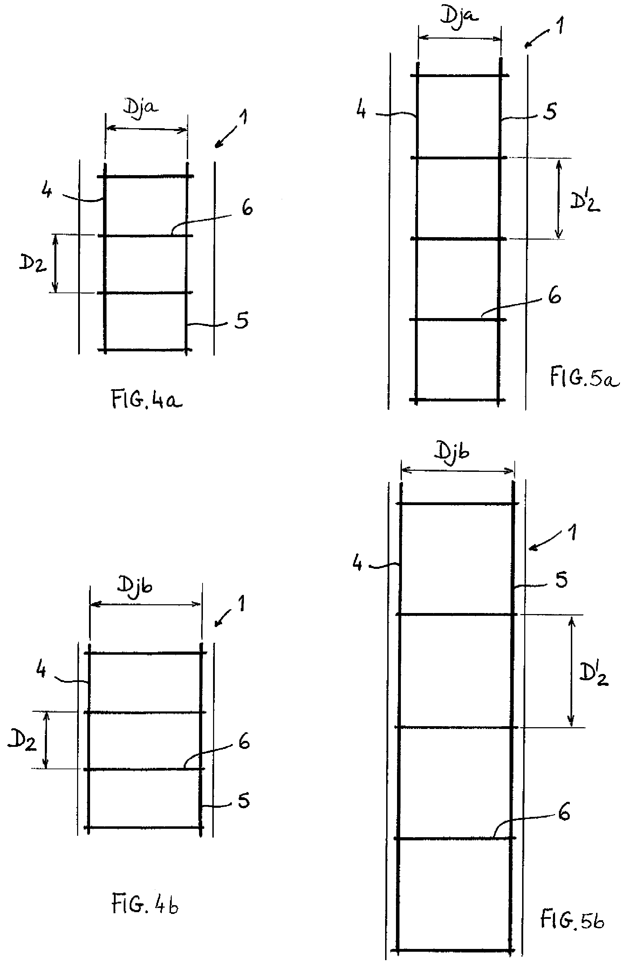

On se reporte à présent aux figures 4a à 5b.

L'invention prévoit de fournir une gamme comprenant plusieurs bandes 1 dont les lignes transversales 6 sont espacées d'une même distance D2 d'une bande à l'autre (à l'état non contraint), mais dont les lignes longitudinales 4, 5 sont plus ou moins espacées d'une bande à l'autre.

Dans l'exemple, on a représenté une première bande (figure 4a) dont les lignes longitudinales sont espacées d'une distance Dja, et une deuxième bande (figure 4b) dont les lignes longitudinales sont espacées d'une distance Djb supérieure à Dja.

Les figures 5a et 5b montrent les bandes illustrées respectivement sur la figure 4a et sur la figure 4b, étirées chacune avec la traction appropriée, c'est-à-dire pour qu'on obtienne des motifs carrés.

Comme on le voit sur les figures 4a à 5b, plus les lignes longitudinales 4, 5 sont écartées (et pour une même distance D2 séparant les lignes transversales 6), plus il faudra étirer la bande 1 pour obtenir des motifs carrés. La valeur de la tension T sera donc plus élevée avec la deuxième bande de la figure 4b qu'avec la première bande de la figure 4a. Ainsi, on pourra obtenir une même compression P pour des patients dont le rayon R de cheville est différents, et ce en utilisant l'une ou l'autre des bandes 1 de la gamme.

A titre d'exemple, une gamme peut notamment comporter les deux bandes suivantes :
- première bande :
   D1 = 42,9 mm
   D2 = 33,0 mm
   allongement nécessaire pour l'obtention de carrés : 30% tension obtenue avec cet allongement approprié : T = 50 cN/cm soit pour un rayon R de cheville de 3,66 cm (circonférence de 23 cm) une pression P de 13,66 cN/cm²
- deuxième bande :
   D1 = 46,2 mm
   D2 = 33,0 mm
   allongement nécessaire pour l'obtention de carrés : 40%
   tension obtenue avec cet allongement approprié : T = 60 cN/cm soit pour un rayon R de cheville de 4,30 cm (circonférence de 27 cm) une pression P de 13,95 cN/cm²

Ainsi, pour deux patients ayant des rayons de cheville différents, on peut obtenir des compressions sensiblement identiques grâce à la gamme selon l'invention, qui propose des bandes dont les lignes longitudinales sont plus ou moins écartées.

Il va de soi que l'invention n'est pas limitée au mode de réalisation décrit ci-dessus à titre d'exemple mais qu'elle en embrasse au contraire toutes les variantes de réalisation.

## Revendications

1. Bande de contention comprenant des motifs (7) dont la dimension longitudinale est inférieure à la dimension transversale à l'état non contraint de la bande (1), la dimension longitudinale pouvant être rendue sensiblement égale à la dimension transversale lorsqu'une traction longitudinale adéquate est exercée sur la bande (1), **caractérisée en ce que** les motifs sont formés par :
- deux lignes longitudinales (4, 5) s'étendant sur sensiblement toute la longueur de la bande (1), espacées d'une distance D1, ces lignes longitudinales (4, 5) étant constituées chacune d'au moins un fil de chaîne d'une couleur distincte de la couleur de la bande (1) ;
- et une pluralité de lignes transversales (6), reliant les deux lignes longitudinales (4, 5), ces lignes transversales (6) étant sensiblement régulièrement espacées d'une distance D2 inférieure à D1, à l'état non contraint de la bande (1), et présentant une couleur distincte de la couleur de la bande (1).

2. Bande de contention selon la revendication 1, **caractérisée en ce que** les lignes transversales (6) s'étendent de part et d'autre des deux lignes longitudinales (4, 5).

3. Bande de contention selon la revendication 1 ou 2, **caractérisée en ce que** les lignes transversales (6) sont imprimées.

4. Bande de contention selon la revendication 1 ou 2, **caractérisée en ce que** les lignes transversales (6) sont obtenues par un effet d'armure.

5. Bande de contention selon la revendication 1 ou 2, **caractérisée en ce que** les lignes transversales (6) sont constituées par au moins un fil de trame.

6. Bande de contention selon l'une des revendications 1 à 5, **caractérisée en ce qu'**elle présente, selon sa direction longitudinale, plusieurs portions, chaque portion comportant une pluralité de lignes transversales sensiblement régulièrement espacées d'une distance Di inférieure à D1, les distances Di variant d'une portion à l'autre de la bande (1).

7. Gamme **caractérisée en ce qu'**elle comprend N bandes de contention selon l'une des revendications 1 à 6, où N ≥ 2, les lignes longitudinales (4, 5) d'une bande (1) donnée étant espacées d'une distance Dj, les distances Dj étant différentes pour toutes les bandes (1) de la gamme, et les lignes transversales (6) étant espacées d'une distance D2, la distance D2 étant identique pour toutes les bandes (1) de la gamme et inférieure à toutes les distances Dj des bandes (1) de la gamme.

8. Gamme **caractérisée en ce qu'**elle comprend N bandes de contention selon l'une des revendications 1 à 6, où N ≥ 2, les lignes longitudinales (4, 5) d'une bande (1) donnée étant espacées d'une distance D1, la distance D1 étant identique pour toutes les bandes (1) de la gamme, et les lignes transversales (6) étant espacées d'une distance Dk, les distances Dk étant différentes pour toutes les bandes (1) de la gamme et inférieures à D1.

## Patentansprüche

1. Kompressionsbandage, Muster (7) umfassend, deren Längsabmessung im nicht gespannten Zustand der Bandage (1) kleiner ist, als die Querabmessung, wobei die Längsabmessung der Querabmessung in etwa gleichgestellt werden kann, wenn ein entsprechender Zug in Längsrichtung auf die Bandage (1) ausgeübt wird, **dadurch gekennzeichnet, dass** die Muster gebildet werden durch:
- zwei Längslinien (4, 5), die sich in einem Abstand D1 über etwa die gesamte Länge der Bandage (1) erstrecken, wobei diese Längslinien (4, 5) jeweils durch zumindest einen Kettfaden in einer Farbe gebildet werden, die sich von der Farbe der Bandage (1) unterscheidet;
- und eine Vielzahl von Querlinien (6), welche die beiden Längslinien (4, 5) miteinander verbinden, wobei diese Querlinien (6) in etwa regelmäßig in einem Abstand D2 zueinander angeordnet sind, der im nicht gespannten Zustand der Bandage (1) kleiner als D1 ist, und eine Farbe aufweist, die sich von der Farbe der Bandage (1) unterscheidet.

2. Kompressionsbandage nach Anspruch 1, **dadurch gekennzeichnet, dass** sich die Querlinien (6) beiderseits der beiden Längslinien (4, 5) erstrecken.

3. Kompressionsbandage nach Anspruch 1 oder 2, **dadurch gekennzeichnet, dass** die Querlinien (6) aufgedruckt sind.

4. Kompressionsbandage nach Anspruch 1 oder 2, **dadurch gekennzeichnet, dass** man die Querlinien (6) durch einen Bindungseffekt erhält.

5. Kompressionsbandage nach Anspruch 1 oder 2, **dadurch gekennzeichnet, dass** die Querlinien (6) durch zumindest einen Schussfaden gebildet werden.

6. Kompressionsbandage nach einem der Ansprüche 1 bis 5, **dadurch gekennzeichnet, dass** sie in ihre Längsrichtung mehrere Abschnitte aufweist, wobei jeder Abschnitt eine Vielzahl von Querlinien umfasst, die in etwa regelmäßig in einem Abstand Di zueinander angeordnet sind, der kleiner als D1 ist, wobei die Abstände Di von einem Abschnitt der Bandage (1) zum anderen variieren.

7. Produktreihe, **dadurch gekennzeichnet, dass** sie N Kompressionsbandagen nach einem der Ansprüche 1 bis 6 umfasst, wobei N ≥ 2 ist, wobei die Längslinien (4, 5) einer gegebenen Bandage (1) in einem Abstand Dj zueinander angeordnet sind, wobei die Abstände Dj für alle Bandagen (1) der Produktreihe unterschiedlich sind, und die Querlinien (6) in einem Abstand D2 zueinander angeordnet sind, wobei der Abstand D2 für alle Bandagen (1) der Produktreihe gleich ist, und kleiner als alle Abstände Dj der Bandagen (1) der Produktreihe.

8. Produktreihe, **dadurch gekennzeichnet, dass** sie N Kompressionsbandagen nach einem der Ansprüche 1 bis 6 umfasst, wobei N ≥ 2 ist, wobei die Längslinien (4, 5) einer gegebenen Bandage (1) in einem Abstand D1 zueinander angeordnet sind, wobei der Abstand D1 für alle Bandagen (1) der Produktreihe gleich ist, und die Querlinien (6) in einem Abstand Dk zueinander angeordnet sind, wobei die Abstände Dk für alle Bandagen (1) der Produktreihe unterschiedlich sind, und kleiner als D1.

## Claims

1. A compression band comprising patterns (7) whose longitudinal dimension is lower than the transverse dimension at the unconstrained state of the band (1), the longitudinal dimension can be made substantially equal to the transverse dimension when an adequate longitudinal traction is exerted on the band (1), **characterized in that** the patterns are formed by:
- two longitudinal lines (4, 5) extending over substantially the entire length of the band (1), spaced apart by a distance D1, these longitudinal lines (4, 5) being consisting each of at least one warp yarn with a different color from the band (1) color;
- and a plurality of transverse lines (6), connecting the two longitudinal lines (4,5), these transverse lines (6) being substantially regularly spaced apart by a distance D2 lower than D1, at the unconstrained state of the band (1), and having a different color from the color of the band (1).

2. The compression band according to claim 1, **characterized in that** the transverse lines (6) extend on either side of the two longitudinal lines (4, 5).

3. The compression band according to claims 1 or 2, **characterized in that** the transverse lines (6) are printed.

4. The compression band according to claims 1 or 2, **characterized in that** the transverse lines (6) are obtained by a weave effect.

5. The compression band according to claims 1 or 2, **characterized in that** the transverse lines (6) are constituted by at least one weft yarn.

6. The compression band according to any of claims 1 to 5 **characterized in that** it has, along its longitudinal direction, several portions, each portion including a plurality of transverse lines substantially regularly spaced apart by a distance Di lower than D1, the distances Di varying from one portion to the other of the band (1).

7. A range **characterized in that** it comprises N compression bands according to any of claims 1 to 6, where N ≥ 2, the longitudinal lines (4, 5) of a given band (1) being spaced apart by a distance Dj, the distances Dj being different for all the bands (1) of the range, and the transverse lines (6) being spaced apart by a distance D2, the distance D2 being identical for all the bands (1) of the range and lower than all the distances Dj of the bands (1) of the range.

8. The range **characterized in that** it comprises N compression bands according to any of claims 1 to 6, where N ≥ 2, the longitudinal lines (4, 5) of a given band (1) being spaced apart by a distance D1, the distance D1 being identical for all the bands (1) of the range, and the transverse lines (6) being spaced apart by a distance Dk, the distances Dk being different for all the bands (1) of the range and lower than D1.
